# EUROPEAN PATENT APPLICATION

(11) **EP 4 088 730 A1**
(43) Date of publication of application: **16.11.2022**
(21) Application number: 22173091.4
(22) Date of filing: 12.05.2022
(51) Int. Cl.: A61K 36/15, A23L 33/105, A61P 11/00, A61P 11/04, A61P 31/04

(54) **ANTIMICROBIAL COMPOSITITON FOR THE TREATMENT OF THE UPPER RESPIRATORY TRACT INFECTIONS**

(30) Priority: 13.05.2021 IT 202100012344
(71) Applicant: Unifarco S.p.A., 32035 Santa Giustina (BL) (IT)
(72) Inventor: Baratto, Giovanni, 32035 Santa Giustina (IT); Riva, Ernesto, 32035 Santa Giustina (IT); Ferrari, Sara, Santa Giustina Santa Giustina (IT)
(74) Representative: Perani & Partners S.p.A.

(57) **Abstract**

*Dry extract of Larix decidua* for use in the treatment and prevention of bacterial infections of the upper respiratory tract.

## Description

### FIELD OF THE INVENTION

The present invention relates to an extract for use in the treatment of upper respiratory tract conditions, obtained from the industrial waste of *Larix decidua* (European larch) wood processing.

### BACKGROUND ART

Larch is an important tree at an industrial level, characterised by a strong, waterproof and durable wood. Barks and branches are considered waste material of the wood industry but are important sources of biologically active compounds, even if only partially explored at present. Several coniferous barks contain phytoconstituents, such as tannins, terpenes and polyphenols. Many studies have also considered these plant materials mainly for their essential oil composition. In recent years, this Applicant has investigated the possibility of extracting phytochemicals from the bark; in particular the content of polyphenolic derivatives and as a potential source of antioxidants has been evaluated.

Larch wood is strong, waterproof and durable, but also pliable when cut into thin strips. Heartwood is particularly resistant to the elements and is therefore mainly used as construction timber. Due to its high resin content and difficult workability, its use in furniture manufacturing is limited.

Larch sawdust is a by-product of larch wood production. Currently, larch sawdust is mainly used for the production of fuel pellets and contains phenolic compounds, such as lignans and flavonols (mainly dihydroquercetin and dihydrokaempferol). Arabinogalactans are constituents of larch and are a class of long, densely branched polysaccharides with molecular weights comprised between 10,000 and 120,000 Da.

Arabinogalactan larch is considered a good source of dietary fibre and has been approved as such by the FDA. It also has potential therapeutic benefits as an immunostimulatory agent and as an adjuvant to the anti-cancer protocol.

Larch also has many traditional uses, as an anthelmintic, a diuretic, a laxative and a cicatrizant, for example.

Larch bark extract was considered to possess diuretic properties, and powdered bark was used as a topical application to promote the healing of purulent wounds and also in chronic eczema and psoriasis. A resinous material rich in terpenes can be extracted from the bark of *Larix decidua,* and Venice turpentine is thus obtained. In traditional European medicine, Venice turpentine is used to formulate preparations for internal and external disorders that are used for kidney disease, as an antiseptic and as an expectorant in chronic bronchitis. It is important to use only small quantities, as larger amounts can cause kidney damage (internally) and blister swelling (externally).

A study published by Baldan et al. [Molecules 2017] discusses an extraction process from *Larix decidua,* a deciduous conifer endemic of Europe, naturally occurring throughout central Europe from the Alps, in eastern France, through the Carpathian Mountains, Slovenian Mountains, to southern Poland, western Ukraine and northern Romania.

In the work of Baldan et al., larch bark was used to prepare extracts using environmentally friendly solvents. The extracts obtained were characterised by the content of polyphenols and proanthocyanidins (PAC) in order to evaluate the antioxidant activity of the extract.

The findings of Baldan et al indicated that larch bark is a valuable source of antioxidant compounds such as flavonoids and type B proanthocyanidins (PACs), and that it can be an innovative and sustainable ingredient for pharmaceutical, nutraceutical and cosmetic purposes.

The antimicrobial properties of an extract in methanol/water from the bark of conifers have been described in the literature. However, for nutraceutical and cosmetic purposes it is not possible to use extracts obtained by extraction in methanol.

### SUMMARY OF THE INVENTION

The Applicant has surprisingly discovered that it is possible to use a standardised dried extract of *Larix decidua* from larch bark obtained by hydroalcoholic extraction.

This extract has been shown to exhibit antimicrobial activity against several pathogens of the upper respiratory tract, including *Staphylococcus aureus, Streptococcus pneumoniae, Klebsiella pneumoniae* and *Pseudomonas aeruginosa,* commonly spread, demonstrating that larch bark could represent a novel antimicrobial ingredient for pharmaceutical or nutraceutical formulations.

It is therefore an object of the present invention to provide oral pharmaceutical or nutraceutical compositions comprising a *Larix decidua* bark extract as an active ingredient for use in the treatment and prevention of bacterial infections of the upper respiratory tract.

### DESCRIPTION OF THE FIGURES

Figure 1 shows the HPLC-FLD chromatogram of larch bark proanthocyanidins. The
Figure illustrates the peaks corresponding to higher grade monomers, dimers and oligomers.
Figure 2 shows Type A proanthocyanidins in dried larch bark powder (left panel) and larch bark extract (right panel).
Figure 3 shows Type B proanthocyanidins in dried larch bark powder (left panel) and larch bark extract (right panel).
Figure 4 shows the percentage of inhibition of bacterial growth of respiratory pathogens treated with larch bark extract and Grapefruit Seed Extract (GSE) (*Citrus paradisi*) at different concentrations (200-20-2 µg/mL). The values represent the means of the measurements, made in triplicate (n = 3). The bars represent the standard deviation. GSE: ^{∗} p <0.05; ^{∗∗} p <0.01; ^{∗∗∗} p <0.001 vs bacteria treated with larch bark extract.

### DETAILED DESCRIPTION OF THE INVENTION

For the purposes of the present invention, the definitions "comprising" or "containing" provide for the possibility of further components in addition to those mentioned after said definition.

Conversely, for the purposes of the present invention the definition "consisting of" excludes the possibility of further components other than those listed after said definition.

For the purposes of the present invention, by nutraceutical is meant the definition given by Stephan Defelice in 1989, i.e. a food, or part thereof, preferably a part thereof, (specifically the extract that is the object of the present invention) and which has positive effects for well-being and health, including the prevention and treatment of diseases.

By nutraceutical composition is meant an oral formulation containing said nutraceutical in combination with suitable excipients and/or diluents.

By pharmaceutical composition is meant preferably an oral formulation containing the extract that is the object of the present invention as an active pharmaceutical ingredient in combination with suitable excipients and/or diluents.

By cosmetic composition is meant a formulation, preferably topical, containing the aforementioned extract as active ingredient in combination with suitable excipients and/or diluents.

In this work, a standardised Larch Bark Extract (LBE) obtained from organic bark waste, in particular from the industrial waste of larch wood processing, was developed using an industrial pilot plant, with the aim of evaluating this product as a natural antimicrobial ingredient for pharmaceutical and nutraceutical applications. To this end, its antibacterial activity against respiratory pathogens such as *Staphylococcus aureus, Streptococcus pneumoniae, Klebsiella pneumoniae, Pseudomonas aeruginosa, Haemophilus influenza, and Streptococcus pyogenes* has been studied and compared with a commercial grapefruit (Citrus paradisi) seed extract (GSE), a raw material widely proposed in recent years as an antimicrobial ingredient for cosmetic formulations and food supplements.

The *Larix decidua* dry extract is obtained from the bark and is preferably obtained from the industrial waste of the *Larix decidua* wood processing.

The dry extract of *Larix decidua* is obtained by extraction with a hydroalcoholic solvent, preferably ethanol, with a water content comprised preferably between 30 and 70, more preferably ethanol is present at 40%.

The extraction process comprises the following steps:
a) the larch bark is dried until a final moisture content of less than 12% is obtained;
b) the bark is ground to obtain a powder with D50 comprised between 250 and 350 µm, preferably 300 µm;
c) the material contained in the ground product from stage b) is extracted in a hydroalcoholic solution at room temperature for a time comprised between 50 and 70 minutes, preferably 60 minutes;
d) the solvent is removed preferably by centrifugation, vacuum concentration and oven drying.

The dried product obtained in step d) is sifted until a D50 comprised between 200 and 300 µm is obtained, preferably 250µm.

The dry extract of *Larix decidua* for use according to the present invention is therefore in the form of a powder having D50 comprised between 200 and 300 µm, preferably less than 250 µm.

The dry extract of *Larix decidua* for use according to the present invention preferably has a total proanthocyanidins content comprised between 8% and 17%, more preferably 15% by weight on the total weight.

The dry extract of *Larix decidua* has a PAC-A/PAC-B ratio comprised between 0.07 and 0.13, preferably 0.10.

*Larix decidua* dry extract is indicated for use in the treatment and prevention of bacterial infections of the upper respiratory tract, in particular those induced by *Staphylococcus aureus, Streptococcus pneumoniae, Klebsiella pneumoniae,* and *Pseudomonas aeruginosa.*

The following experimental examples are provided solely for the purposes of illustrating the extract extraction process for use according to the present invention, the results of the HPLC-FLD-MS spectrometric analyses of the extract obtained by such a process and, finally, the evaluation of the antimicrobial activity of the extract for use according to the present invention against bacteria of the upper respiratory tract.

### Example 1- Extraction procedure

The larch bark was dried at 40°C for 40 hours to a final humidity of 5%. The dried bark material was ground to a fine particle size (d = 300 µm) and extracted using a mixture of 40 ethanol/60% water under stirring for 60 min at room temperature, with a powder: solvent ratio of 1:10 w/w.

The extract was then centrifuged using an industrial centrifuge and concentrated under vacuum using a concentrator. Finally, the solvent was removed to dryness using a dryer cabinet. The dried extract was then ground and sifted to obtain a particle size specification of less than 250 µm.

### Example 2-HPLC-FLD-MS Analysis of Larch Bark PACs

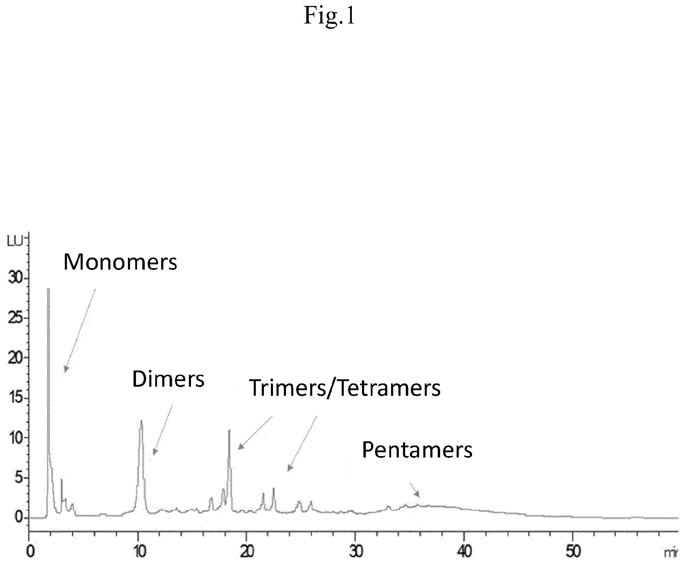

The total amount of PAC-A and PAC-B in LBE and their degree of polymerisation were assessed by HPLC-FLD-MS in both LBE and dried larch bark powder. Based on MS data, the chromatogram was divided into four parts, corresponding to the monomers (PAC units flavan-3-ols consisting of catechins or epicatechins) and the different polymer classes PAC (dimer-hexamers) **(****Figure 1****).** The total amount of PAC in LBE has a heterogeneous polymer class composition and is about 16% (six times) higher than dried larch bark powder. Table 1 shows in detail the different amounts of polymeric classes of PAC in the dried powder of larch bark and in the extract of larch bark, expressed as % w/w obtained by HPLC-FLD analysis.

**Table 1**

| **Sample of larch** | **PACs % w/w** | | | | |
|---|---|---|---|---|---|
| | **Mon** | **Dim** | **Tri/Tetr** | **Pent/Esa** | **Total** |
| Dried larch bark | 0.93±0.07 | 0.36±0.03 | 0.38±0.06 | 0.91±0.01 | **2.58** |
| Extract of *L. decidua* | 5.12±0.10 | 3.21±0.06 | 2.32±0.18 | 5.28±0.24 | **15.92** |

| | | | | | |
|---|---|---|---|---|---|
| Mon: monomers; Dim: dimers; Tri: trimers; Tetr: tetramers; Pent: pentamers; Esa: hexamers. | | | | | |

MS analysis was performed to distinguish between type A and type B PAC and specific *m*/*z* values were monitored. Epicatechin was monitored at m/z 289. For dimer-tetramers with one or more ether bonds corresponding to PAC type A, [MH] - m/z ions 575 were monitored for dimers, m/z 863 for trimers, m/z 1149 and m/z 1151 for tetramers and m/z 1437 and m/z 1727 for pentamers and hexamers, respectively.

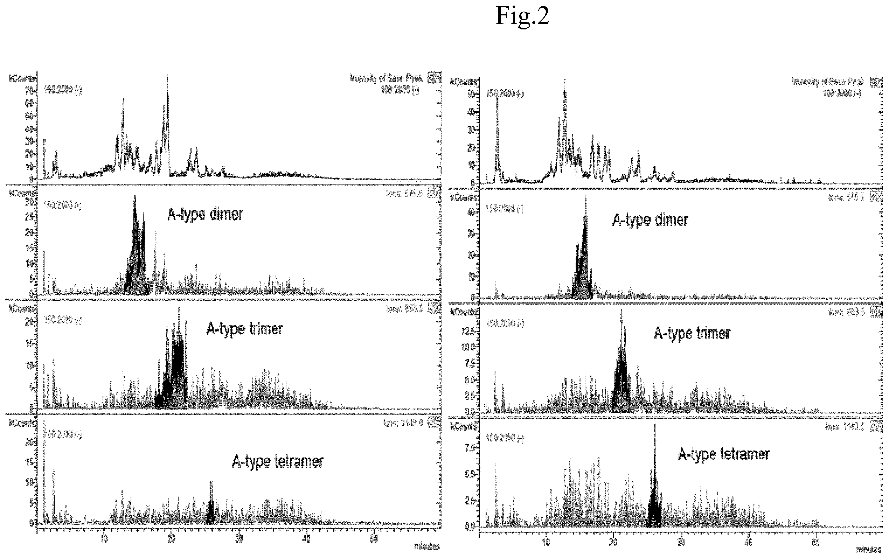

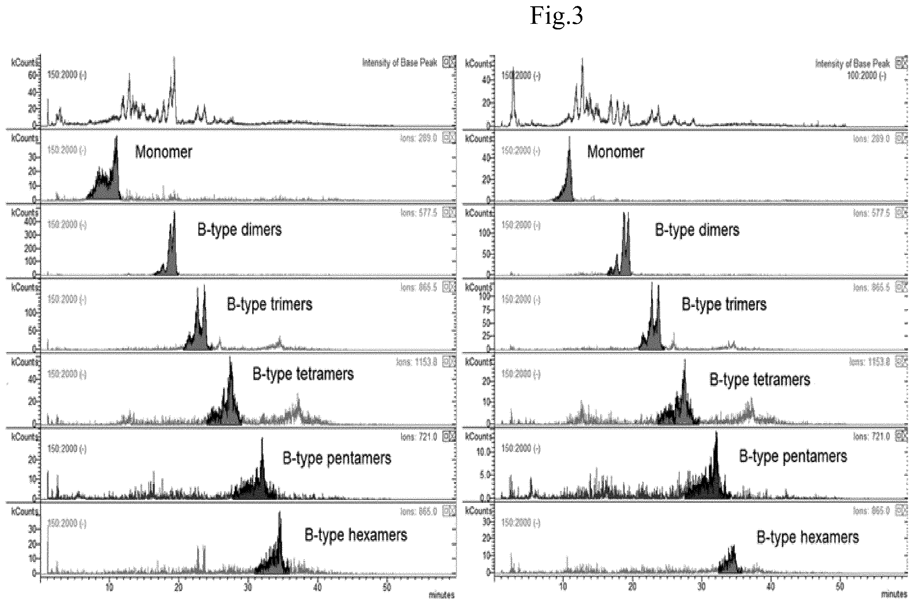

For those PACs without ethereal bonds, corresponding to type B PACs, [MH]- ions at m/z 577, m/z 865 and m/z 1153, were monitored respectively for dimers, trimers and tetramers, while [M -2H] 2- ions at m/z 721 and 865 were monitored respectively for pentamers and hexamers. The relative percentages of PAC-A and PAC-B oligomers and PAC-A / PAC-B ratios of dried larch bark powder and LBE are given in Table 2, while the respective HPLC-MS chromatograms are given in Figures 2 and 3.

**Table 2: PAC-A and PAC-B% and PAC-A/PAC-B relative ratio of the degree of polymerisation of PAC contained in dried larch bark powder and larch bark extract.**

| **Sample of larch** | **Degree of polymerisa tion** | **Relative %** | | | **PAC-A/PAC-B ratio** |
|---|---|---|---|---|---|
| | | **PAC-A** | **PAC-B** | **PAC-A+PAC-B** | |
| Dried larch bark | Dimers | 10.29±0.23 | 33.55±0. 63 | 43.83±0.86 | 0.31±0.00 |
| | Trimers | 3.38±0.09 | 32.69±0. 11 | 36.07±0.21 | 0.10±0.00 |
| | Tetramers | 0.88±0.05 | 9.14±0.0 6 | 10.02±0.01 | 0.09±0.01 |
| | Pentamers | - | 4.81±0.0 8 | 4.81±0.08 | - |
| | Hexamers | - | 4.68±0.0 8 | 4.68±0.08 | - |
| | **Total %** | **14.60** | **85.40** | **100.00** | 0.17 |
| Extract of *L. decidua* | Dimers | 5.03±0.10 | 49.00±0. 02 | 54.02±0.08 | 0.10±0.00 |
| | Trimers | 3.72±0.10 | 23.19±0. 10 | 26.92±0.21 | 0.16±0.00 |
| | Tetramers | 0.52±0.01 | 8.89±0.0 4 | 9.41±0.03 | 0.06±0.00 |
| | Pentamers | - | 4.79±0.0 2 | 4.79±0.02 | - |
| | Hexamers | - | 4.76±0.0 2 | 4.76±0.02 | - |
| | **Total %** | **9.28** | **90.72** | **100.00** | 0.10 |

The most evident difference between the larch bark powder and the extract in the matter of composition PAC-A and PAC-B lies in the relationship between the two types of PAC (PAC-A: PAC-B oligomers = 0.17 in larch bark powder; 0.10 in larch bark extract). The higher amount of PAC-A in the dried powder is mainly due to the higher percentage of dimers compared to the extract (Table 2). On the other hand, the relative percentage of PAC-B dimers appears to be higher in LBE, while PAC-B trimers are present at a lower percentage. Oligomers with higher molecular weights appear to be comparable between the larch bark powder and the extract (Table 2). The abundance of PAC in LBE could partly explain the antibacterial activity observed.

### Example 3-Assessment of antimicrobial activity against respiratory pathogens

The antimicrobial activity of the extract of *L. decidua* (LBE) was determined by the microplate dilution technique. Bacteria were grown in appropriate broth *(Staphylococcus aureus Methicillin-Resistant* (MRSA), *Klebsiella pneumoniae* and *Pseudomonas aeruginosa* in Tryptic Soy Broth (TSB); *Streptococcus pyogenes* in Brain Heart Infusion (BHI) supplemented with 10% v/v Fetal Bovine Serum (FBS); *Streptococcus pneumoniae* in thioglycolate broth, *Haemophilus influenzae* type B (HIB) supplemented with NAD and Emina) at 37°C. After 16 h they were harvested by centrifugation and an Optical Density (OD) bacterial suspension of 0.5 McFarland (1.5 x 108 Colony Forming Unit (CFU)/mL) was prepared. 100 µl of properly diluted bacteria were then inoculated into 96-well microplates. Stock solutions of LBE and GSE were prepared and added to the microplates in a concentration range of 2-200 µg / mL. The microplates were incubated at 37°C with a slight movement. Optical Density at 620 nm was recorded at baseline and then after 16 hours of incubation to evaluate the effect of extracts on bacterial growth.

The percentage of "survivors" was calculated relative to the control mixture without any extract.

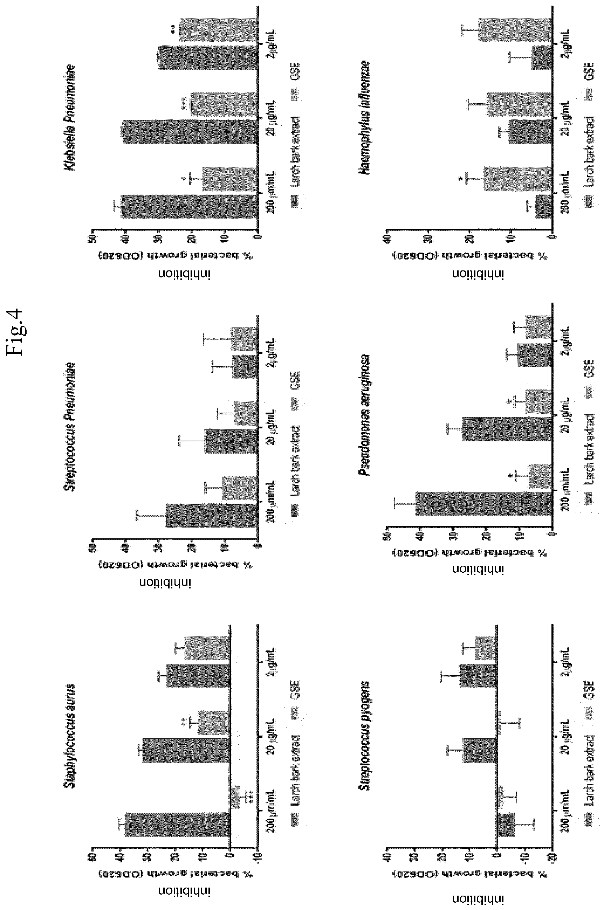

The results of the antimicrobial tests on respiratory pathogens are given in Figure 4. With regard to antimicrobial activity on Methicillin-Resistant Staphylococcus Aureus (MRSA), LBE inhibited growth by 38.13% and 31.87% at the concentration of 200 and 20 µg / ml, respectively, while by 21.1% at 2 µg/ml. At 2 and 20 µg / mL, GSE showed lower anti-MRSA activity than LBE, while at the higher dose GSE slightly increased bacterial growth.

Focusing on *Streptococcus pneumoniae,* LBE significantly inhibited pathogen growth at 200 µg/mL (22% inhibition) with greater efficiency than GSE (10.6% inhibition). LBE at 20 µg/mL maintained a moderate growth inhibition activity (16%), while at 2 µg/mL the inhibitory activity was barely detectable (7% inhibition). However, GSE lacked significant inhibitory activity as early as 20 µg/mL.

Considering *Klebsiella pneumoniae,* LBE demonstrated significant growth inhibitory capacity at all concentrations tested, reducing pathogen growth by 41.2%, 40.9%, and 30%, respectively. GSE reduced bacterial growth to a significantly lower level (by 17-23%).

LBE significantly inhibited *Pseudomonas aeruginosa* growth at 200 µg/mL (42%) and 20 µg/mL (28%), while at 2 µg/mL modest activity (10%) was detected. In contrast, the GSE showed no significant antimicrobial activity against *Pseudomonas aeruginosa* at all concentrations tested.

Considering *Haemophilus influenzae,* LBE did not significantly affect microbial growth at any tested concentration, whereas GSE showed moderate inhibitory activity (15%) at all tested doses. Dose-response correlation was observed for neither extract. Both LBE and GSE did not demonstrate significant antimicrobial activity on *Streptococcus pyogenes* at any tested concentration.

The dry extract of *Larix decidua* thus obtained may be contained in an oral composition in combination with suitable excipients and/or diluents and any further active ingredients, wherein said oral composition is a pharmaceutical or nutraceutical composition.

## Claims

1. Dry extract of *Larix decidua* for use in the treatment and prevention of bacterial infections of the upper respiratory tract.

2. Dry extract for use according to claim 1, wherein said bacterial infections are induced by *Staphylococcus aureus, Streptococcus pneumoniae, Klebsiella pneumoniae and Pseudomonas aeruginosa.*

3. Dry extract of *Larix decidua* for use according to claim 1 or 2, wherein said extract is obtained from the industrial waste of the processing of *Larix decidua* wood.

4. Dry extract of *Larix decidua* for use according to claim 3, wherein said dry extract is obtained by extraction with a hydroalcoholic solvent, preferably ethanol, with a water content comprised between 30 and 70, preferably 60% by weight.

5. Dry extract of *Larix decidua* for use according to any one of claims 1-4, wherein said extract is in the form of a powder having an average diameter D50 comprised between 200 and 300 µm, preferably less than 250µm.

6. Dry extract of *Larix decidua* for use according to any one of claims 3-5, having a total proanthocyanidins titre comprised between 8 and 17%, preferably 15% by weight on the total weight.

7. Dry extract of *Larix decidua* for use according to claim 5, wherein the PAC-A/PAC-B content is comprised between 0.07 and 0.13, preferably it is 0.10.

8. Dry extract of *Larix decidua* for use according to any one of claims 1-7, wherein said extract is contained in an oral composition in combination with suitable excipients and/or diluents and any further active ingredients.

9. Dry extract of *Larix decidua* for use according to claim 8, wherein said oral composition is a pharmaceutical or nutraceutical composition.
